Europäisches Patentamt

European Patent Office

Office européen des brevets

(19)

(11) Publication number: 0 090 443

B1

(12) EUROPEAN PATENT SPECIFICATION

(45) Date of publication of patent specification: 22.06.88

(21) Application number: 83200336.2

(22) Date of filing: 09.03.83

(51) Int. Cl.⁴: C 07 C 51/12, C 07 C 53/08,
C 07 C 67/36, C 07 C 69/14,
B 01 J 31/18, B 01 J 31/30

(54) A process for carbonylating methanol to acetic acid and/or methyl acetate.

(30) Priority: 30.03.82 US 363698

(43) Date of publication of application:
05.10.83 Bulletin 83/40

(45) Publication of the grant of the patent:
22.06.88 Bulletin 88/25

(84) Designated Contracting States:
BE DE FR GB NL

(56) References cited:
EP-A-0 011 043
EP-A-0 055 618
EP-A-0 072 055
DE-B-1 767 150
GB-A-2 089 803

(73) Proprietor: SHELL INTERNATIONALE
RESEARCH MAATSCHAPPIJ B.V.
Carel van Bylandtlaan 30
NL-2596 HR Den Haag (NL)

(72) Inventor: Van Leeuwen, Petrus Wilhelmus N.
Badhuisweg 3
NL-1031 CM Amsterdam (NL)

(74) Representative: Aalbers, Onno et al
P.O. Box 302
NL-2501 CH The Hague (NL)

Courier Press, Leamington Spa, England.

## Description

Carbonylation processes for the preparation of carboxylic acids from alcohols particularly for the production of acetic acid from methanol are well known in the art. The prior art teaches the use of a number of catalysts for the synthesis of carboxylic acids from alcohols and carbon monoxide at elevated temperatures and pressures. Catalysts such as phosphoric acid, phosphates, heavy metal salts such as zinc and cuprous chlorides, silicates of various metals, and boron trifluoride in various hydration states have been reported to function for the production of acetic acid by the reaction of methanol and carbon monoxide at elevated temperatures and pressures of the order to 400°C and 700 bar, respectively. However, even under such severe conditions, the yields of acid were substantially poor, and, therefore, uneconomical. Other catalysts that have been reported under somewhat less severe conditions but still relatively high temperature and pressures are liquid phosphoric acid containing copper phosphate, activated charcoal impregnated with phosphoric acid, and metal carbonyls, such as iron, cobalt and nickel, in conjunction with their halides or free halogenes in liquid phase. Even with the use of these catalysts temperatures and pressures are more severe than would be desired in a commercial operation. A rhodium based catalyst system used in conjunction with a halide promoter has been disclosed in the prior art as allowing for the carbonylation of methanol to acetic acid to occur at relatively low pressures and temperatures. Rhodium, however, is a relatively expensive catalytic material.

More specifically from the published European patent application No. 0,011,043, such a carbonylation process is known, comprising the reaction of methanol with a mixture of hydrogen and carbon monoxide representing at least 75 mol%, at a temperature of the order of 180°C or more, in the presence of an effective quantity of cobalt, at least an ionic iodide and at least a covalent halide, whereby the iodide cation is selected from the group consisting of alkali metal cations, alkaline earth metal cations, ammonium cations, quaternary ammonium cations, phosphonium cations and quaternary phosphonium cations, whereby the ratio between $I^-$/Co (in gr. atoms) is $\geq$ 10 and the ratio $X/I^-$ (in gr. atoms), wherein X represents a halogen atom occurring in a covalent halide, being between 1 and 30%.

On the other hand from the German Auslegeschrift No. 1,767,150 such a carbonylation process is known, comprising the conversion of the starting alcohol with carbon monoxide in the liquid phase at 100—240°C or in the gas phase at 200—240°C and under a carbon monoxide pressure of from 0.35—210 atm, in the presence of a catalyst selected from compounds of the noble metals iridium, platinum, palladium, osmium and/or ruthenium and with a promoter, consisting of bromine, iodine or bromo or iodo-compounds.

Disadvantages of such processes are however the unattractive low conversion rates (giving rise to rather large reactors), which are obtained at the desired relative low temperatures and pressures.

Therefore, there is still a need for a process using a catalytic material which would be much less expensive than rhodium, but would carbonylate methanol to acetic acid at relative low temperatures and pressure with an economical conversion rate.

The present invention relates to a process for carbonylating methanol to acetic acid and/or methyl acetate characterized in that methanol is reacted with carbon monoxide in the presence of a catalyst comprising a catalytically active amount of a homogeneous palladium compound, a promoter RZ wherein R is H or $C_1$—$C_3$ alkyl and Z is chlorine, bromine or iodine and a Group IA-, IIA- or transition metal iodide or carboxylate, and one or more ligands containing at least one electron-rich N atom. Palladium is very active at relatively low temperatures and pressures. It is further more considerably less expensive than corresponding rhodium catalysts by a factor of about 6.

The catalyst can be prepared in several ways. It can be prepared by combining the above components and then charging the resultant catalytic material to the reactor or it can be prepared by charging the individual components to the reactor and allowing the catalyst to form in situ.

The palladium is conveniently provided as a palladium salt which will be soluble in the reaction medium. Suitable palladium salts, are for example, palladium dichloride, palladium acetate, palladium nitrate, palladium sulphate, palladium diiodide, palladium dibromide, and palladium acetylacetonate. The preferred palladium salt is palladium acetate. The palladium need only be present in the reaction medium in small concentrations. Preferred concentrations will range from $10^{-5}$ to $10^{-1}$ moles per litre.

A two-component promoter is utilized, the first component comprising a halide of the general formula RZ wherein R is hydrogen or a lower alkyl having a carbon number ranging from 1 to 3 and Z is chlorine, bromine and/or iodine, and second component comprising a Group IA (alkali metal), IIA (alkaline earth metal) or transition metal iodide or carboxylate. In the absence of metal salts it is determined that the catalyst becomes inactive by plating out of the palladium. Large amounts of the salts, however, can procedure a lessening in rates. Molar ratios of promoter to palladium compound typically are greater than 1:1, preferably greater than about 10:1. Different metal iodide or carboxylate salts will require different optimum ratios of RZ to metal iodide or carboxylate salts. These optimum ratios can readily be determined by routine experimentation.

Preferably the first component will be comprised of an iodide component. Z thus is preferably iodine. Generally, it is preferred that the amount of iodine material present in the reaction medium will be in amounts such that the atomic ratio of iodine to the atomic ratio of palladium is above 2:1. Preferably, the atomic ratio of the iodine to palladium will be in a range of 5:1 to 5000:1. A more preferred atomic ratio of the iodine to palladium is about 10:1 to about 2500:1.

The multi-component catalytic system used in the process of the instant invention also utilizes one or more ligands containing at least one electron-rich nitrogen atom capable of forming coordination compounds with the palladium moieties present in the catalytic system. Examples of ligands which can be used conveniently comprise pyrrole, alkyl substitued pyrroles, pyrrolidine, alkyl-substituted pyrrolidines, pyridine, alkyl-substituted pyridines, piperidines, alkyl-substituted piperidines, pyrimidine, alkyl-substituted pyrimidines, pyrazine, benztriazole, tetra-ethylenediamine, phenanthrolenes such as 1,10-phenanthroline, alkyl-substituted 1,10-phenanthrolines, morpholine and alkyl-subsituted morpholines.

The amount of organo-nitrogen ligand to be used in the process according to the present invention is generally related to the amount of palladium (and transition) metal compound present in the reaction mixture. For example, 2 or more mols of ligand per mol of palladium compound are sufficient. Generally, the molar amounts of ligand present will range from 2 to 10 or higher times the total molar amount of palladium compound present.

The ligands listed in Table I below have been experimentally determined to exert lignad activity. Other ligands containing at least one electron-rich N atom would also be suitable.

TABLE I
Ligands containing at least one electron-rich N atom:

2,2'-bipyridine (Bip)
2,2'-biquinoline
pyridil (=di-2-pyridilglyoxal)
phenanthroline
picolinic acid
2,6-pyridine disulphonic acid (PDSA)
pyrrole
2-quinolinol
picolylmethylamine
N,N,N',N'-tetramethylethylenediamine (TMEDA)
phenylenediamine
pyrrolidinone
ethylenediamine (EDA)
2-aminopyridine
pyridine

A particularly desirable ligand is 2,2'-bipyridine. The concentration of 2,2'-bipyridine is preferably maintained at 2 or more moles of bipyridine per mole of palladium compound. Lower concentrations of bipyridine lower the rates. Thus, the preferred molar ratio of bipyridine to palladium compound ranges from about 2:1 to about 6:1. The 2,2'-bipyridine can be readily made from pyridine by refluxing on Raney nickel with good yield. Crude bipyridine, containing mixtures of 2,2'-, 2,4'- and 4,4'-isomers, can be utilized in the instant invention as a source of 2,2'-bipyridine without separation of the respective isomers.

The carbonylation reaction is carried out by intimately contacting methanol with gaseous carbon monoxide in a liquid phase containing the catalyst system as prepared from the components described above under conditions of temperatures and pressure suitable to form acetic acid and/or methyl acetate. The temperature accordingly will be in the range of 125°C to 250°C, preferably from 165°C to 225°C and most preferably from 175°C to 205°C. Partial pressures of carbon monoxide of the order of 1 bar to 360 bar are suitably employed, preferably, pressures ranging from 20 bar to 210 bar carbon monoxide partial pressure are generally preferred. Higher pressures may be used if desired under appropriate conditions.

The carbon monoxide required for the reaction may be supplied in substantially pure form, or diluted with any inert gas, for example nitrogen. The presence of more than minor amounts of hydrogen in the gas stream has undesirable effects upon the activity of the catalyst. Generally, it is preferred that the amount of hydrogen in the gas stream should be less than about 5% by volume, preferably less than about 1% by volume.

A separate solvent is not essential in the process according to the instant invention, and often a large excess of methanol, acetic acid or methyl acetate forms a convenient reaction medium. However, it may in some cases be desirable to use a separate solvent, and any suitable inert medium may be used. A suitable solvent may, for example, be selected from sulphones, for example dimethyl sulphone, diethyl sulphone or diisopropylsulphone or sulfolanes, either unsubstituted or alkyl substituted such as 2-methyl sulfolane or 3-methylsulfolane.

The following examples are provided to illustrate the invention and are not to be construed as limiting the invention.

Examples
In general, the following examples were carried out in a 300 ml stirred autoclave. The autoclaves was charged with the appropriate reactants as indicated in Table II. The runs were conducted at constant pressure using a high-pressure regulator and a reservoir autoclave filled with CO. Both reaction vessel and reservoir were equipped with a pressure transducer enabling the measurement of the constant pressure in the reaction vessel and the pressure drop in the reservoir. This latter pressure drop is a measure for the rate of reaction. Before starting, the reservoir was pressured up to 270—360 bar and the reaction vessel was charged at room temperature with approximately half the carbon monoxide pressure desired at 170°—100°C. This procedure was followed in order to prevent plating out of metals at low or zero carbon monoxide pressure. The space-time yields are calculated from yields as found by GLC analysis. In cases where certain variables were changed during a run a different procedure was followed. The space-time yield for a particular part of the reaction was calculated from the pressure drop per hour, the overall yield and the overall-pressure drop in the reservoir. The data for these experiments are shown in Table II below. The results are indicated as space-time yields in kilogram per liter per hour and the data also includes a percentage of the conversion over which the space-time yield has been calculated.

TABLE II.
Carbonylation of methanol with Pd

| Example | Pd(OAc)₂ g | Bip g | MeOH g | MeOAc g | H₂O g | CH₃I g | NaI g | Temp °C | Pressure bar | Conv. % | Space-time yield, kg/l h | Remarks |
|---|---|---|---|---|---|---|---|---|---|---|---|---|
| 1 | 0,75 | 1,1 | 24 | 12 | 8 | 7,5 | 3 | 195—225 | 110—140 | 0—123[b] | 0,18[a] | Glass-lining. Temperature overshot. |
| 2 | 0,075 | 0,22 | 24 | 12 | 8 | 7,5 | 3 | 190—200 | 200—140 | 0—117 | 0,19[a] | Conversion too high. 3 runs. |
| 3 | 0,10 | 0,14 | 80 | 40 | 30 | 29 | 7 | 178 | 110 | 0—72 | 0,14 | 2,5 h/run |
| 4 | 0,10 | 0,07 | 80 | 40 | 30 | 29 | 7 | 180 | 110 | 0—44 | 0,07 | 3 h |
| 5 | 0,10 | 0,14 | 80 | 40 | 30 | 22 | 14 | 180 | 110 | 0—54 | 0,12 | 2 h |
| 6 | 0,20 | 0,28 | 80 | 40 | 30 | 50 | 20 | 182 | 110 | 0—53 | 0,26 | 1 h/run |
| 7 | 0,25 | 0,035 | 80 | 40 | 30 | 25 | 13 | 192 | 140 | 0—8 | 0,12 | |
| | | | | | | | | | | 8—15 | 0,16 | Added 0,025 g Pd acetate 0.035 g Bip |
| | | | | | | | | | | 15—30 | 0,18 | Added 0,050 g Pd acetate 0.070 g Bip |
| | | | | | | | | | | 0—30 | 0,16 | Average |
| 8 | 0,10 | 0,14 | 80 | 40 | 30 | 25 | 13 | 192 | 140 | 0—12 | 0,34 | |
| | | | | | | | | | | 12—25 | 0,35 | Added 0.10 g Pd acetate 0.14 g Bip |
| | | | | | | | | | | 35—78 | 0,08 | Added 0.20 g Pd acetate, 0.28 g Bip |
| | | | | | | | | | | 0—78 | 0,15 | Average |

[a] Based on an assumed 60—70% liquid volume in autoclave.
[b] Yields of greater than 100% refer to conversion of solvent methyl acetate in addition to reactant methanol.

Using a general procedure similar to that described above, the carbonylation of methanol was carried out in an 80 ml autoclave using different organo-nitrogen ligands. The autoclave was initially charged with 50 bar CO pressure at 25°C. The reaction was carried out at 300°C for 3 hours. The results are shown in Table III.

TABLE III
Carbonylation of methanol using various ligands

| Pd(OAc)$_2$ catalyst (g) | Ligand (g) | Methanol (g) | CH$_3$I (g) | Sulfolane (g) | Salt (g) | Carbonylation % |
|---|---|---|---|---|---|---|
| 0,005 | TMEDA[a) 0,17 | 4 | 1,5 | 14 | NiI$_2$ 0,17 | 75 |
| 0,005 | PDSA[b) 0,20 | 4 | 1,5 | 14 | NiBr$_2$ 0,10 | 58 |
| 0,005 | BIP[c) 0,10 | 2 | 0,75 | 7 | FeI$_2$ 0,10 | 38 |
| 0,005 | 2-amino-pyrdine 0,2 | 4 | ˙1,5 | 14 | Ni(OAc)$_2$· 4H$_2$0 0,10 | 62 |

[a) N,N,N'-N'-tetramethylethylenediamine
[b) 2,6-pyridine disulphonic acid
[c) 2,2'-bipyridine

**Claims**

1. A process for carbonylating methanol to acetate acid and/or methyl acetate characterized in that methanol is reacted with carbon monoxide in the liquid phase, at a temperature in the range of 125°C to 250°C and under a partial pressure of carbon monoxide in the range of 1 bar to 360 bar, in the presence of a catalyst comprising: a catalytically active amount of a homogeneous palladium compound, a promoter RZ wherein R is H or C$_1$—C$_3$-alkyl and Z is chlorine, bromine or iodine and a Group IA, IIA or transition metal iodide or carboxylate, and one or more ligands containing at least one electron-rich N atom.

2. A process according to claim 1 characterized in that the ligand is 2,2'-bipyridine, 2,2'-biquinoline, di-2-pyridilglyoxal, phenanthroline, picolinic acid, 2,6-pyridine disulphonic acid, pyrrole, 2-quinolinol, picolylmethylamine, N,N,N',N'-tetramethylethylenediamine, phenylenediamine, pyrrolidinone, ethylenediamine, 2-aminopyridine, or pyridine.

3. A process according to claims 1 or 2 characterized in that the palladium compound concentration ranges from 10$^{-5}$ to 10$^{-1}$ moles per litre, the molar ratio of ligand to palladium compound is 2:1 or more, the atomic ratio of Z to palladium ranges from 5:1 to 5000:1, the molar ratio of second promoter to palladium compound is greater than 1:1.

4. A process according to claim 1 characterized in that the temperature ranges from 165°C to 225°C.

5. A process according to claims 1—4 characterized in that the temperature ranges from 175°C to 205°C and the pressure ranges from 20 bar to 210 bar.

**Patentansprüche**

1. Verfahren zur Carbonylierung von Methanol zu Essigsäure und/oder Methylacetat, dadurch gekennzeichnet, daß Methanol mit Carbonmonoxid in flüssiger Phase bei einer Temperatur im Bereich von 125 bis 250°C und unter einem Partialdruck von 1 bis 360 bar, in Gegenwart eines Katalysators, umfassend eine katalytisch wirksame Menge einer homogenen Palladiumverbindung, einen Beschleuniger RZ, wobei R Wasserstoff oder eine C$_1$—C$_3$-Alkylgruppe und Z Chlor, Brom oder Iod ist, und ein Iodid oder Carboxylat eines Elements der Gruppe IA, IIA oder eines Übergangsmetalls und einen oder mehrere Liganden, die mindestens eine elektronenreiches (stark elektronenanziehendes) N-Atom enthalten, umsetzt.

2. Verfahren nach Anspruch 1, dadurch gekennzeichnet, daß der Ligand 2,2'Bipyridin, 2,2'Bichinolin, Di-2-pyridylglyoxal, Phenanthrolin, Picolinsäure, 2,6-Pyridin-disulfonsäure, Pyrrol, 2-Chinolinol, Picolylmethylamin, N,N,N',N'-Tetramethylethylendiamin, Phenylendiamin, Pyrrolidinon, Ethylendiamin, 2-Aminopyridin oder Pyridin ist.

3. Verfahren nach einem der Ansprüche 1 oder 2, dadurch gekennzeichnet, daß die Konzentration an Palladiumverbindung im Bereich von 5:1 bis 5000:1 liegt und das Verhältnis des zweiten Beschleunigers zu Palladium größer als 1:1 ist.

6

4. Verfahren nach Anspruch 1, dadurch gekennzeichnet, daß die Temperatur im Bereich von 165 bis 225°C liegt.

5. Verfahren nach einem der Ansprüche 1 bis 4, dadurch gekennzeichnet, daß die Temperatur im Bereich von 175 bis 205°C und der Druck im Bereich von 20 bis 210 bar liegt.

**Revendications**

1. Un procédé pour la carbonylation de méthanol en acide acétique et/ou en acétate de méthyle, caractérisé en ce que le méthanol est mis à réagir avec l'oxyde de carbone dans la phase liquide, à une température comprise entre 125°C et 250°C et sous une pression partielle d'oxyde de carbone comprise entre 1 bar et 360 bars, en présence d'un catalyseur comprenant: une quantité catalytiquement active d'un composé du palladium homogène, un promoteur RZ où R est H ou un groupe alcoyle en $C_1$—$C_3$ et Z est du chlore, du brome ou de l'iode et un iodure ou carboxylate d'un métal du groupe IA ou IIA ou d'un métal de transition, et un ou plusieurs ligands contenant au moins un atome d'azote riche en électrons.

2. Un procédé selon la revendication 1, caractérisé en ce que le ligands est la 2,2'-bipyridine, la 2,2'-biquinoléine, le di-2-pyridylglyoxal, la phénanthroline, l'acide picolinique, l'acide 2,6-pyridine disulfonique, le pyrrole, le 2-quinolinol, la picolylméthylamine, la N,N,N',N'-tétraméthyléthylènediamine, la phénylènediamine, la pyrrolidinone, l'éthylènediamine, la 2-aminopyridine ou la pyridine.

3. Un procédé selon les revendications 1 ou 2, caractérisé en ce que la concentration du composé du palladium est comprise entre $10^{-5}$ et $10^{-1}$ mole par litre, le rapport molaire du ligand du composé du palladium est de 2:1 ou plus, le rapport atomqiue de Z au palladium est compris entre 5:1 et 5000:1, le rapport molaire du second promoteur au composé du palladium est supérieur à 1:1.

4. Un procédé selon la revendication 1, caractérisé en ce que la température est comprise entre 165°C et 225°C.

5. Une procédé selon les revendications 1—4, caractérisé en ce que la température est comprise entre 175°C et 205°C et la pression est comprise entre 20 bars et 210 bars.